# EUROPEAN PATENT SPECIFICATION

(11) **EP 3 829 548 B1**
(45) Date of publication and mention of the grant of the patent: **17.06.2026**
(21) Application number: 19844270.9
(22) Date of filing: 31.07.2019
(51) Int. Cl.: A61K 31/765, A61K 39/39, A61K 9/51, A61K 9/14, A61K 47/69, C12N 5/09, A61P 35/00, A61K 45/06, A61K 9/16

(54) **IMMUNE MODIFYING PARTICLES FOR THE TREATMENT OF CANCER**
IMMUNMODIFIZIERENDE PARTIKEL ZUR BEHANDLUNG VON KREBS
PARTICULES MODIFIANT LA RÉPONSE IMMUNITAIRE DESTINÉES AU TRAITEMENT DU CANCER

(30) Priority: 31.07.2018 US 201862712604 P; 04.01.2019 US 201962788569 P
(43) Date of publication of application: 09.06.2021
(73) Proprietor: Cour Pharmaceuticals Development, Co., Inc., Skokie, IL 60077 (US)
(72) Inventor: PUISIS, John, Northbrook, IL 60062 (US)
(74) Representative: CMS Cameron McKenna Nabarro Olswang LLP
(86) International application number: PCT/US2019/044454
(87) International publication number: WO 2020/028544

(56) References cited:
- WO-A2-2014/160465
- US-A1- 2006 034 925
- US-A1- 2015 010 631
- YOSHIKO IWAI ET AL: "Cancer immunotherapies targeting the PD-1 signaling pathway", JOURNAL OF BIOMEDICAL SCIENCE, vol. 24, no. 1, 4 April 2017 (2017-04-04), XP55649210, DOI: 10.1186/s12929-017-0329-9
- MILES A. MILLER ET AL: "Tumour-associated macrophages act as a slow-release reservoir of nano-therapeutic Pt(IV) pro-drug", NATURE COMMUNICATIONS, vol. 6, no. 1, 27 October 2015 (2015-10-27), XP055682738, DOI: 10.1038/ncomms9692
- YAN F ET AL: "The effect of poloxamer 188 on nanoparticle morphology, size, cancer cell uptake, and cytotoxicity", NANOMEDICINE: NANOTECHNOLOGY, BIOLOGY, AND MEDICINE, ELSEVIER, AMSTERDAM, NL, vol. 6, no. 1, 1 February 2010 (2010-02-01), pages e170 - e178, XP026882573, ISSN: 1549-9634, [retrieved on 20090515], DOI: 10.1016/J.NANO.2009.05.004
- JINGWEI XIE ET AL: "Self-Assembled Biodegradable Nanoparticles Developed by Direct Dialysis for the Delivery of Paclitaxel", PHARMACEUTICAL RESEARCH, KLUWER ACADEMIC PUBLISHERS-PLENUM PUBLISHERS, NL, vol. 22, no. 12, 1 December 2005 (2005-12-01), pages 2079 - 2090, XP019370754, ISSN: 1573-904X, DOI: 10.1007/S11095-005-7782-Y
- QIAN CHEN ET AL: "Photothermal therapy with immune-adjuvant nanoparticles together with checkpoint blockade for effective cancer immunotherapy", NATURE COMMUNICATIONS, vol. 7, no. 1, 21 October 2016 (2016-10-21), pages 1 - 13, XP055537345, DOI: 10.1038/ncomms13193
- KOSMIDES A K ET AL: "Biomimetic biodegradable artificial antigen presenting cells synergize with PD-1 blockade to treat melanoma", BIOMATERIALS, ELSEVIER, AMSTERDAM, NL, vol. 118, 2 December 2016 (2016-12-02), pages 16 - 26, XP029860654, ISSN: 0142-9612, DOI: 10.1016/J.BIOMATERIALS.2016.11.038
- MILLER, MA ET AL.: "Tumour-Associated Macrophages act as a Slow-Release Reservoir of Nano-Therapeutic Pt(IV) Pro-Drug", NATURE COMMUNICATIONS, vol. 6, no. 8692, 27 October 2015 (2015-10-27), pages 1 - 13, XP055682738

## Description

### FIELD OF THE DISCLOSURE

The present disclosure relates, in general, to methods of treating cancer and proliferative diseases using immune-modifying particles in combination with checkpoint inhibitors by altering myeloid-derived suppressor cells (MDSCs), tumor-associated macrophages (TAMs), neutrophils, and monocytes in the tumor microenvironment.

### BACKGROUND

Distinct myeloid-derived cell populations are now widely recognized as being part of the tumor microenvironment. These cells include monocytes, tumor associated macrophages (TAMs), myeloid-derived suppressor cells (MDSCs) and dendritic cells (Kumar et al., Trends Immunol., 37(3):208-220 (2016); Richards et al., Cancer Microenviron., 6(2):179-91 (2013)). A significant body of work suggests that these cells are actively recruited to tumor sites and sites of metastasis where they can be altered by cues from the local milieu to promote an immunosuppressive microenvironment (Kumar et al., Trends Immunol.,37(3):208-220 (2016); Kitamura et al., Front Immunol., 8:2004 (2018); Kitamura et al., J Exp Med., 212(7):1043-59 (2015)). The immunosuppressive activity of these cells has been shown to promote tumor growth, proliferation, angiogenesis, metastasis, and tumor immune evasion. Furthermore, presence of MDSCs and TAMs in the tumor microenvironment (TME) is correlated with poor disease outcomes (Gabrilovich et al., Nat Rev Immunol.,12(4):253-68 (2012); Ouzounova et al., Nat Comm., 8:14979 (2017); Marvel et al J Clin Invest.,125(9): 3356-64 (2015)).

In addition to immune cells, the tumor stroma plays a role in shaping the tumor microenvironment and influencing tumor growth and progression. Cellular and molecular constituents of the tumor-associated stroma including fibroblasts, mesenchymal stromal cells, adipocytes, endothelium, and the extracellular matrix (ECM) have all been shown to contribute to tumorigenesis (Valkenburg et al., Nat Rev Clin Oncol., 15(6):366-381 (2018)). Tissue-resident and bone marrow mesenchymal stem cell (MSC)-derived cancer associated fibroblasts (CAFs) have been shown to secrete growth factors and proteins that alter anti-tumor immune responses and promote tumor growth and metastasis (Valkenburg et al., Nat Rev Clin Oncol., 15(6):366-381 (2018); Shiga et al., Cancers, 7,2443-2548 (2015)). Similarly, ECM proteins, mesenchymal stromal cells, endothelial cells, and adipocytes have been reported to dampen anti-tumor immunity and facilitate tumor progression (Lu et al., J Cell Biol., 196(4):395-406 (2012); Kumar et al., Cancer Cell 32(5):654-668.e5 (2017); Quante et al., Cancer Cell., 19, 257-272 (2011); Park et al., Endocr Rev., 32(4):550-70 (2011); Young et al., Cancer Immunol Immunother., 61(10):1609-16 (2012); Hida et al., Int J Mol Sci., 19(5):1272 (2018)).

While significant progress has been made in developing novel anti-cancer therapeutics, the efficacy of these therapies has shown limited promise due to the fact that they target tumors but not immune suppressive factors in the tumor microenvironment (TME) that inhibit anti-tumor immune function and promote tumor progression.

Signaling through immune checkpoint receptors (e.g., Programmed cell death protein 1 (PD-1) and CTLA-4) and their ligands (e.g., PD-L1) regulates the activity of cytotoxic T-cells and has been shown to play a critical role in modulating inflammatory immune responses. Importantly, numerous tumor types are known to hijack the PD-1/PD-L1 and CTLA-4 immune checkpoint signaling pathways in order to evade the T-cell-mediated anti-tumor immune responses. Thus, targeting of immune checkpoint signaling pathways, using specific inhibitors like monoclonal antibodies, has emerged as an attractive frontline treatment option in numerous cancers (Alsaab et al., Front Pharmacol 8:561 (2017)). However, the presence of myeloid-derived suppressor cells (MDSCs) and Tumor-associated macrophages (TAMs) in the tumor microenvironment along with the tumor-associated stroma have been shown to dampen the anti-tumor efficacy of immune checkpoint inhibitors like anti-PD1 monoclonal antibodies (Weber et al., Front Immunol.,9:1310, (2018); Highfill et al., Sci Transl Med., 6(237):237ra67 (2014); Zhao et al., Cancer Immunol Res., 6(12):1459-1471 (2016); Wang et al., Nat Commun., 9(1):3503 (2018); Yoshiko Iwai et al., Journal of Biomedical Science, vol. 24(1), 2017).

Immune modifying Particles (IMPs) are negatively charged nanoparticles that have immunomodulatory properties (See e.g. US Patent Publication Nos. US20150010631; US 20130323319).

### SUMMARY

The present invention is defined by the claims. Any subject-matter falling outside the scope of the claims is provided for information only.

Methods of treatment of the human body by therapy, referred to in this description, are not part of the present invention as such, but are described here in relation to compounds and pharmaceutical compositions for use in said methods of treatment of the human body by therapy, according to the present inventio.

According to a first embodiment, the present invention relates to negatively charged poly (lactic-co-glycolic acid) (PLGA) particles for use in a method of treating cancer in a subject, the method comprising administering the negatively charged particles in combination with a cancer therapeutic, wherein the negatively charged particles are biodegradable particles that are free of associated peptides or antigenic moieties or other bioactive agents, wherein the cancer therapeutic is an anti-PD-1 antibody.

According to a second embodiment, the present invention relates to a cancer therapeutic for use in a method of treating cancer in a subject, the method comprising administering negatively charged poly (lactic-co-glycolic acid) (PLGA) particles in combination with the cancer therapeutic, wherein the negatively charged particles are biodegradable particles that are free of associated peptides or antigenic moieties or other bioactive agents, wherein the cancer therapeutic is an anti-PD-1 antibody.

Without being bound by theory, it is hypothesized that IMPs alter immunosuppressive monocyte-derived cells in the tumor microenvironment, which in turn will boost the efficacy of other cancer therapies when administered to the subject. It is suggested herein that combination therapies using IMPs and cancer therapeutics that can target both tumor cells and overcome the immunosuppressive tumor microenvironment by targeting MDSCs, TAMs, neutrophils, other monocyte-derived cells, and the tumor-associated stroma may be able to provide enhanced therapeutic benefit compared to monotherapy with cancer therapeutics, such as immune checkpoint inhibitors.

In various embodiments, the combination therapy according to the present invention, alters the population of myeloid-derived suppressor cells (MDSCs), tumor-associated macrophages (TAMs), neutrophils, monocytes, dendritic cells, and/or stroma at the tumor site.

In various embodiments, the combination therapy according to the invention alters the tumor-associated stroma.

In various embodiments, the combination therapy according to the invention alters stromal connective tissue, fibroblasts, endothelium, adipose tissue, extracellular matrix, pericytes, cancer stem cells, mesenchymal stem cells, and/or mesenchymal stromal cells.

In various embodiments, the combination therapy according to the invention induces tumor cell death, apoptosis, and/or necrosis via direct particle uptake by tumor cells.

In various embodiments, the combination therapy according to the invention regulates the anti-tumor immune response.

In various embodiments, the combination therapy according to the invention alters or modulates the tumor-specific immune response.

In various embodiments, the combination therapy according to the invention alters the population of myeloid-derived suppressor cells (MDSCs), tumor-associated macrophages (TAMs), dendritic cells, neutrophils and/or monocytes at the tumor site.

In various embodiments, the combination therapy according to the invention alters the tumor-associated stroma.

In various embodiments, the combination therapy according to the invention alters stromal connective tissue, fibroblasts, endothelium, adipose tissue, extracellular matrix, pericytes, mesenchymal stem cells, and/or mesenchymal stromal cells.

In various embodiments, the combination therapy according to the invention reduces tumor size and/or tumor growth.

In various embodiments, the combination therapy according to the invention regulates an anti-tumor immune response.

In various embodiments, the combination therapy according to the invention regulates a tumor-specific immune response.

In various embodiments, combination therapy according to the invention egulates cancer stem cells.

The negatively charged particles according to the present invention are poly (lactic-co-glycolic acid) (PLGA) particles as defined in the claims. In various embodiments, the particle comprises about 50:50, about 80:20 to about 100:0 polylactic acid : polyglycolic acid or from about 50:50, about 80:20 to about 100:0 polyglycolic acid: polylactic acid. In various embodiments, the particle comprises 50:50 polylactic acid: polyglycolic acid. In various embodiments, the particle comprises polylactic acid : polyglycolic acid from about 99:1 to about 1:99, including all values and ranges that lie in between these values.

In various embodiments, the particles have a zeta potential between -100 mV and -1 mV. In various embodiments, the particles have a zeta potential between -80 mV and -30 mV. In some embodiments, the zeta potential of the particle is from about -100 mV to about -40 mV, from about -75 mV to about -40 mV, from about -70 mV to about -30 mV, from about -60 mV to about -35 mV, or from about -50 mV to about -40 mV. In various embodiments, the zeta potential is about -30 mV, -35 mV, -40 mV, -45 mV, -50 mV, -55 mV, -60 mV, -65 mV, -70 mV, - 75 mV, -80 mV, -85 mV, -90 mV, -95 mV or -100 mV, including all values and subranges that lie between these values.

In various embodiments, the diameter of the negatively charged particle is between 0.1 µm to 10 µm. In various embodiments, the particle has an average diameter of between about 0.2 µm and about 2 µm; between about 0.3 µm to about 5 µm; between about 0.5 µm to about 3 µm; or between about 0.5 µm to about 1 µm. In some embodiments, the particle has a diameter of about 100 to 1500 nm, about 200 to 2000 nm, about 100 to 10000 nm, about 300 to 1000 nm, about 400 to 800 nm, or about 200 to 700 nm. In various embodiments, the particle has an average diameter of about 100nm, 200 nm, 300 nm, 400nm, 500 nm, 600 nm, 700 nm, 800 nm, 900 nm, 1000 nm, 1100 nm, 1200 nm, 1300 nm, 1400 nm, 1500nm, or 2000 nm, including all values and subranges that lie between these values. In some embodiments, the diameter of the negatively charged particle is between 400 nm to 800 nm.

In various embodiments, the subject has a cancer selected from the group consisting of brain cancer, skin cancer, eye cancer, breast cancer, prostate cancer, pancreatic cancer, lung cancer, esophageal cancer, head and neck cancer, cervical cancer, liver cancer, colon cancer, colorectal cancer, rectal cancer, bone cancer, uterine cancer, ovarian cancer, bladder cancer, stomach cancer, oral cancer, thyroid cancer, kidney cancer, testicular cancer, leukemia, lymphoma and mesothelioma. Additional cancers contemplated by the methods are disclosed in the Detailed Description. various embodiments, the anti-PD-1 antibody according to the present invention is an antibody selected from the group consisting of pembrolizumab and nivolumab.

In various embodiments, the particle and/or the cancer therapeutic is administered twice weekly, once weekly, once every two weeks, once every three weeks, once every 4 weeks, once every two months, once every three months, once every 6 months or once per year.

In various embodiments, the particle and/or the cancer therapeutic is administered intravenously, orally, nasally, intramuscularly, ocularly, transdermally, or subcutaneously.

In various embodiments, the subject is a mammal. In various embodiments, the subject is human.

In various embodiments, the administration improves one or more symptoms of the cancer. In various embodiments, the one or more symptoms are selected from the group consisting of tumor size or tumor burden in the subject, tumor metastasis, and levels of inflammatory cells in the tumor or in the tumor microenvironment. In various embodiments, the administration reduces the tumor size or tumor burden by 10%, 20%, 30% or more. In various embodiments, the administration alters monocytes, macrophages, granulocytes, dendritic cells and/or neutrophils at the tumor.

In various embodiments, the particle is formulated in a composition comprising a pharmaceutical acceptable carrier, diluent or excipient. In various embodiments, the cancer therapeutic is formulated in a composition comprising a pharmaceutical acceptable carrier, diluent or excipient. In various embodiments, the particle and cancer therapeutic can be formulated in the same composition or in separate compositions.

### BRIEF DESCRIPTION OF THE DRAWINGS

Figure 1 sets out an example treatment schedule for administration of IMP and checkpoint inhibitor anti-PD-1 antibody.
Figure 2 shows that IMP plus anti-PD-1 combination therapy exhibits improved reduction in tumor growth compared to single therapy treatment in LLC cells.

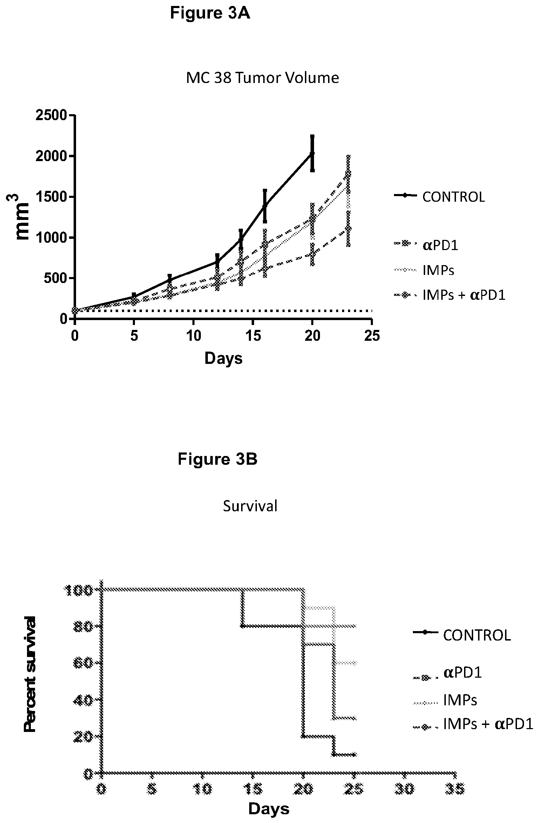

Figure 3A-3B shows the anti-tumor efficacy of IMPs when administered alone or in combination with anti-PD1 checkpoint inhibitor antibody. Mice were implanted with MC38 tumor cells and treated with either saline control, IMPs, anti-PD1, or IMPs + anti-PD1 after palpable tumor formation. (Figure 3A) Compared to control treatment, treatment with IMPs inhibited tumor growth. IMP efficacy was comparable to anti-PD1 treatment. Treatment with IMPs+anti-PD1 demonstrated synergy with an enhanced inhibition of tumor growth. (Figure 3B) Compared to control treatment, treatment with IMPs prolonged survival. IMP survival efficacy was superior to anti-PD1. Treatment with IMPs+anti-PD1 demonstrated synergy with a prolonged survival compared to control and respective monotherapy.

### DETAILED DESCRIPTION

IMPs present an attractive opportunity to specifically target immunosuppressive monocytes in circulation and prevent them from trafficking to tumor sites where their immunosuppressive activities promote tumor growth, proliferation and metastasis. Combining disruption/alteration of the immune suppressive tumor microenvironment by IMP therapy with other anti-cancer therapeutics, like anti-PD1 monoclonal antibodies, is expected to provide significant benefit over monotherapies that target only tumors.

### Definitions:

It is noted here that as used in this specification and the appended claims, the singular forms "a," "an," and "the" include plural reference unless the context clearly dictates otherwise.

"Particle" as used herein refers to any non-tissue derived composition of matter, it may be a sphere or sphere-like entity, bead, or liposome. The term "particle", the term "immune modifying particle", and the term "bead" may be used interchangeably depending on the context. Additionally, the term "particle" may be used to encompass beads and spheres.

"Biodegradable as used herein refers to a particle comprising a polymer that may undergo degradation, for example, by a result of functional groups reacting with the water in the solution. The term "degradation" as used herein refers to becoming soluble, either by reduction of molecular weight or by conversion of hydrophobic groups to hydrophilic groups. Biodegradable particles do not persist for long times in the body, and the time for complete degradation can be controlled. Biocompatible, biodegradable polymers include polymers or copolymers of caprolactones, carbonates, amides, amino acids, orthoesters, acetals, cyanoacrylates and degradable urethanes, as well as copolymers of these with straight chain or branched, substituted or unsubstituted, alkanyl, haloalkyl, thioalkyl, aminoalkyl, alkenyl, or aromatic hydroxy- or di-carboxylic acids. In addition, the biologically important amino acids with reactive side chain groups, such as lysine, arginine, aspartic acid, glutamic acid, serine, threonine, tyrosine and cysteine, or their enantiomers, may be included in copolymers with any of the aforementioned materials to provide reactive groups for conjugating to antigen peptides and proteins or conjugating moieties. Biodegradable materials suitable for the present invention are PLGA polymers.

"Negatively charged particle" as used herein refers to particles which have been modified to possess a net surface charge that is less than zero.

Zeta potential is the charge that develops at the interface between a solid surface and its liquid medium. "Negative zeta potential" refers to a particle having a zeta potential of the particle surface as represented in milliVolts (mV), and measured by an instrument known in the field to calculate zeta potential, e.g., a NanoBrook ZetaPlus zeta potential analyzer or Malvern Zetasizer.

"Carboxylated particles" or "carboxylated beads" or "carboxylated spheres" includes any particle that has been modified or surface functionalized to add one or more carboxyl group onto the particle surface. In some embodiments the addition of the carboxyl group enhances phagocyte/monocyte uptake of the particles from circulation, for instance through the interaction with scavenger receptors such as MARCO. Carboxylation of the particles can be achieved using any compound which adds carboxyl groups, including, but not limited to, Poly (ethylene-maleic anhydride) (PEMA).

The term "regulate" or "alter" as used herein refers to modification, modulation or alteration of an immune response at the tumor site or in the tumor microenvironment. Examples of modifying or altering an immune response include, but are not limited to, decreasing immunosuppressive cell numbers or activity at the tumor site or in the tumor microenvironment, reducing immunosuppressive immune cell infiltration at the tumor site or in the tumor microenvironment, increasing pro-inflammatory immune-cell numbers at the tumor site or tumor microenvironment, increasing pro-inflammatory immune-cell infiltration at the tumor site or tumor microenvironment, increasing pro-inflammatory immune-cell activity or function at the tumor site or tumor microenvironment, increasing anti-tumor immune-cell numbers at the tumor site or tumor microenvironment, increasing anti-tumor immune-cell infiltration at the tumor site or in the tumor microenvironment, increasing the anti-tumor activity or function of immune cells at the tumor site or in the tumor microenvironment, and/or killing of cells associated with immunosuppression.

The term "tumor microenvironment" (TME) as used herein refers to cells, molecules, and blood vessels that surround and feed a tumor cell (National Cancer Institute Dictionary of Cancer Terms). The tumor microenvironment includes immune cells, such as bone-marrow derived inflammatory cells, myelo-monocytic cells, myeloid derived suppressor cells, tumor associated-macrophages, dendritic cells, and lymphocytes, stroma, fibroblasts, signaling molecules and the extracellular matrix (ECM) (Joyce et al., Science 348:74-80, 2015).

"Subject" as used herein refers to a human or non-human animal, including a mammal or a primate, that is administered a particle as described herein. Subjects can include animals such as dogs, cats, rats, mice, rabbits, horses, pigs, sheep, cattle, and humans and other primates.

The term "therapeutically effective amount" is used herein to indicate the amount of target-specific composition of the disclosure that is effective to ameliorate or lessen one or more symptoms or signs of the disease or disorder being treated.

The terms "treat", "treated", "treating" and "treatment", as used with respect to methods herein refer to eliminating, reducing, suppressing or ameliorating, either temporarily or permanently, either partially or completely, one or more clinical symptom, manifestation or progression of an event, disease or condition. Such treating need not be absolute to be useful.

### Checkpoint Modulators

Programmed cell death protein 1 (PD-1), also known as cluster of differentiation 279 (CD279), is a cell surface co-inhibitory receptor expressed on activated T cells, B cells and macrophages, and is a component of immune checkpoint blockade (Shinohara et al., Genomics., 23(3):704, (1994); Francisco et al., Immunol Rev., 236: 219, (2010)). PD-1 limits the activity of T cells upon interaction with its two ligands PD-L1 (also known as B7-H1; CD274) and PD-L2 (B7-DC; CD273) (Postow et al., J Clin Oncol., 33: 9, (2015)). Interaction of PD-1 with PD-L1 and PD-L2, reduces T-cell proliferation, cytokine production, and cytotoxic activity (Freeman GJ et al., J Exp Med., 192:1027-34, (2000); Brown JA et al., J Immunol., 170:1257-66, (2003)).

Two monoclonal antibodies have been approved by the U.S. Food and Drug Administration (FDA) for the inhibition of PD-1 immunotherapy. Pembrolizumab (KEYTRUDA^{®}, Merck Sharp & Dohme Corp.) is approved for use in metastatic melanoma, and nivolumab (Opdivo^{®}, Bristol-Myers Squibb) is approved for use in metastatic melanoma and metastatic squamous non-small cell lung cancer (NSCLC). Both of these antibodies bind to the PD-1 receptor and block its interaction with its ligands, PD-L1 and PD-L2. In various embodiments, the anti-PD-1 antibody inhibits or blocks binding of the PD-1 receptor to one or both of its ligands, PD-L1 and PD-L2.

Additional antibodies to PD-1 have been described in US Patent Nos. 8,735,553; 8,617,546; 8,008,449; 8,741,295; 8,552,154; 8,354,509; 8,779,105; 7,563,869; 8,287,856; 8,927,697; 8,088,905; 7,595,048; 8,168,179; 6,808,710; 7,943,743; 8,246,955; and 8,217,149.

### Immune-Modifying Particles

In some embodiments, the present disclosure provides for a PLGA particle with a negative zeta potential and free of associated antigens, peptides or other bioactive materials for use in a method of treating cancer in combination with an anti-PD-1 antibody.

The particles of the disclosure can be manufactured by any means known in the art. Exemplary methods of manufacturing particles include, but are not limited to, microemulsion polymerization, interfacial polymerization, precipitation polymerization, emulsion evaporation, emulsion diffusion, solvent displacement, and salting out (Astete and Sabliov, J. Biomater. Sci. Polymer Edn., 17:247-289(2006)). Methods of making particles contemplated herein are disclosed in US Patent 9,616,113 and International Patent Publication WO/2017/143346. Manipulation of the manufacturing process for PLGA particles can control particle properties (e.g. size, size distribution, zeta potential, morphology, hydrophobicity/hydrophilicity, polypeptide entrapment, etc). The size of the particle is influenced by a number of factors including, but not limited to, the concentration of polymer, e.g., PLGA, the solvent used in the manufacture of the particle, the nature of the organic phase, the surfactants used in manufacturing, the viscosity of the continuous and discontinuous phase, the nature of the solvent used, the temperature of the water used, sonication, evaporation rate, additives, shear stress, and sterilization.

In various embodiments, the particle is a co-polymer having a molar ratio from about 50:50, about 80:20 to about 100:0 polylactic acid:polyglycolic acid or from about 50:50, about 80:20 to about 100:0 polyglycolic acid:polylactic acid. In some embodiments, the particle is a poly(lactic-co-glycolic acid) particle. In various embodiments, the particle comprises 50:50 polylactic acid: polyglycolic acid. In various embodiments, the particle comprises polylactic acid : polyglycolic acid from about 99:1 to about 1:99, including all values and ranges that lie in between these values.

In some embodiments, the zeta potential of the particle is from about -100 mV to about -1 mV. In some embodiments, the zeta potential of the particle is from about -100 mV to about -40 mV, from about -80 mV to about -30 mV, from about -75 mV to about -40 mV, from about -70 mV to about -30 mV, from about -60 mV to about -35 mV, or from about -50 mV to about -40 mV. In various embodiments, the zeta potential is about -30 mV, -35 mV, -40 mV, -45 mV, -50 mV, -55 mV, -60 mV, -65 mV, -70 mV, -75 mV -80 mV, -85 mV, -90 mV, -95 mV or -100 mV, including all values and ranges that lie in between these values.

In some embodiments, the particle has an average diameter of between about 0.1 µm to about 10 µm. In some embodiments, the particle has an average diameter of between 0.2 µm and about 2 µm. In some embodiments, the particle has a diameter of between about 0.3 µm to about 5 µm. In some embodiments, the particle has a diameter of between about 0.5 µm to about 3 µm. In some embodiments, the particle has a diameter of between about 0.5 µm to about 1 µm. In some embodiments, the particle has a diameter of about 100 to 1500 nm, about 200 to 2000 nm, about 100 to 10000 nm, about 300 to 1000 nm, about 400 to 800 nm or about 200 to 700 nm, including all values and ranges that lie in between these values.

To administer particles as described herein to human or other mammals, the particle may be formulated in a sterile composition comprising one or more sterile pharmaceutically acceptable carriers. The phrase "pharmaceutically or pharmacologically acceptable" refer to molecular entities and compositions that do not produce allergic, or other adverse reactions when administered using routes well-known in the art, as described below. "Pharmaceutically acceptable carriers" include any and all clinically useful solvents, dispersion media, coatings, antibacterial and antifungal agents, isotonic and absorption delaying agents and the like.

Pharmaceutical compositions of the present disclosure containing a particle herein may contain sterile pharmaceutically acceptable carriers or additives depending on the route of administration. Examples of such carriers or additives include water, a pharmaceutical acceptable organic solvent, collagen, polyvinyl alcohol, polyvinylpyrrolidone, a carboxyvinyl polymer, carboxymethylcellulose sodium, polyacrylic sodium, sodium alginate, water-soluble dextran, carboxymethyl starch sodium, pectin, methyl cellulose, ethyl cellulose, xanthan gum, gum Arabic, casein, gelatin, agar, diglycerin, glycerin, propylene glycol, polyethylene glycol, Vaseline, paraffin, stearyl alcohol, stearic acid, human serum albumin (HSA), mannitol, sorbitol, lactose, a pharmaceutically acceptable surfactant and the like. Additives used are chosen from, but not limited to, the above or combinations thereof, as appropriate, depending on the dosage form of the present invention. For solutions or emulsions, suitable carriers include, for example, aqueous or alcoholic/aqueous solutions, emulsions or suspensions, including saline and buffered media. Parenteral vehicles can include sodium chloride solution, Ringer's dextrose, dextrose and sodium chloride, lactated Ringer's or fixed oils. Intravenous vehicles can include various additives, preservatives, or fluid, nutrient or electrolyte replenishers. A variety of aqueous carriers are suitable, e.g., sterile phosphate buffered saline solutions, bacteriostatic water, water, buffered water, 0.4% saline, 0.3% glycine, and the like, and may include other proteins for enhanced stability, such as albumin, lipoprotein, globulin, etc., subjected to mild chemical modifications or the like.

It is contemplated that the particle may further comprise a surfactant. The surfactant can be anionic, cationic, or nonionic. Surfactants in the poloxamer and poloaxamines family are commonly used in particle synthesis. Surfactants that may be used, include, but are not limited to PEG, Tween-80, gelatin, dextran, pluronic L-63, PVA, methylcellulose, lecithin, DMAB and PEMA. Additionally, biodegradable and biocompatible surfactants including, but not limited to, vitamin E TPGS (D-α-tocopheryl polyethylene glycol 1000 succinate). In certain embodiments, two surfactants are used. For example, if the particle is produced by a double emulsion method, the two surfactants can include a hydrophobic surfactant for the first emulsion, and a hydrophobic surfactant for the second emulsion.

Therapeutic formulations of the particle are prepared for storage by mixing the particle having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, succinate and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; or metal complexes (e.g., Zn-protein complexes).

Preparations of particles can be stabilized by lyophilization. The addition of a cryoprotectant such as trehalose can decrease aggregation of the particles upon lyophilization. Any suitable lyophilization and reconstitution techniques can be employed. It will be appreciated by those skilled in the art that lyophilization and reconstitution can lead to varying degrees of antibody activity loss and that use levels may have to be adjusted to compensate.

### Methods of Use

Provided herein is a method of treating cancer in a subject comprising administering a negatively charged particle in combination with a cancer therapeutic, as defined in the claims, wherein said particle is free from attached peptide or antigenic moieties and wherein the administering alters the population of myeloid-derived suppressor cells (MDSCs), tumor-associated macrophages (TAMs), neutrophils, dendritic cells, and/or monocytes at the tumor site.

In various embodiments, the disclosure provides a method of treating a cancer in a subject comprising administering to the subject negatively charged particles in combination with a cancer therapeutic, as defined in the claims, wherein the administering alters the tumor-associated stroma, and/or wherein the administering alters stromal connective tissue, fibroblasts, endothelium, adipose tissue, extracellular matrix, pericytes, mesenchymal stem cells, and/or mesenchymal stromal cells, and/or wherein the administering reduces tumor size and/or tumor growth, and/or wherein the administering regulates an anti-tumor immune response, and/or wherein the administering regulates a tumor-specific immune response.

In various embodiments, the disclosure provides a method of treating a cancer in a subject, comprising administering to the subject negatively charged particles in combination with a cancer therapeutic, as defined in the claims, wherein the administering regulates cancer stem cells.

Methods useful in determining the effects of therapeutics on immune cells include, but are not limited to, microscopic analyses, histological assays, cytological assays, flow cytometry, polymerase chain reaction (PCR), quantitative polymerase chain reaction (qPCR), RNA sequencing (RNA-seq), single-cell RNA sequencing (scRNA-seq), next-generation sequencing, whole-exome sequencing, epigenetic sequencing, ATAC-seq, microarray analysis, and mass cytometry or CyTOF. Biomarkers that can be used, alone or in combination, for the evaluation of immune cells include cell surface markers and secreted proteins. Exemplary biomarkers, include, but are not limited to, CD45, CD3, CD4, CD8, CD25, CD44, CD134, CD252, CD137, CD79, CD39, FOXP3, PD-1, LAG-3, TIM-1, IFN-γ, Granzyme, Perforin, CD11b, CD11c, Ly6C, Ly6G, CD14, CD16, CD80, MARCO, CD68, CD115, CD204, CD205, CD206, CD163, CD103, CD103c, F4/80, PD-L1, PD-L2, Arginase, iNOS, ROS, TNF-α, TGF-β, MHC-I, MHC-II, NK1.1, NKG2D, CD244, Ki67, CD19, CD20, CCR2, CXCR3, CCR4, CCR5, CCR6, CCR7, CCR10, CCL2, CCL5, Cx3CR1, CCL10, ICOS, CD40, CD40L, IL1α, IL1β, IL2, IL4, IL5, IL6, IL8, IL12, IL15, IL17, IL21, IL22, TCRγ/δ, TCRα/β, STAT3, ROR1c, and RORγt.

Cancer stem cells (CSCs) have been described as a subset of cells found within solid and hematologic tumors that are tumorigenic, and capable of self-renewal, differentiation. Several reports have described the importance of CSCs in the pathogenesis of a variety of tumors, tumor relapse after therapy, and development of therapeutic resistance. A number of cell surface markers can be used to distinguish CSCs within solid and hematologic tumors. CSC markers include, but are not limited to, CD19, CD20, CD24, CD34, CD38, CD44, CD90, CD133, Aldehyde dehydrogenase 1, CEACAM-6/CD66c, BMI-1, Connexin 43/GJA1, DLL4, EpCAM/TROP1, GLI-1, GLI-2, Integrins, PON1, PTEN, ALCAM/CD166, DPPIV/CD26, Lgr5, Musashi-1, A20, ABCG2, CD15, Fractalkine, HIF-2α, L1CAM, c-MAF, Nestin, Podoplanin, SOX2, CD96, CD117, FLT3, AFP, CD13, CD90, NF2/Merlin, ABCB5, NGFR, Syndecan-1, Endoglin, STRO-1, and PON1.

Multiple diagnostic tools designed to characterize tumors at the cellular and molecular level are FDA-approved and commercially available. Examples of approved diagnostics include FOUNDATIONONE^{®} CDX, FOUNDATIONONE^{®} LIQUID, FOUNDATIONONE ^{®} HEME, BRACAnalysis CDx, therascreen EGFR RGQ PCR kit, cobase EGFR Mutation Test v2, PD-L1 IHC 22C3 pharmDx, Abbott Real Ti*me* IDH1, MRDx BCR-ABL test, VENTANA ALK (D5F3) CDx Assay, Abbott RealTi*me* IDH2, Praxis Extended RAS Panel, Oncomine Dx Target Test, LeukoStrat CDx FLT3 Mutation Assay, FoundationFocus CDx*BRCA* Assay, Vysis CLL FISH Probe Kit, *KIT* D816V Mutation Detection, *PDGFRB* FISH, cobas KRAS Mutation Test, therascreen KRAS RGQ PCR Kit, FerriScan, Dako c-KIT pharmDx, INFORM Her-2/neu, PathVysion HER-2 DNA Probe Kit, SPOT-LIGHT HER2 CISH Kit, Bond Oracle HER2 IHC System, HER2 CISH pharmDx Kit, INFORM HER2 DUAL ISH DNA Probe Cocktail, HercepTest, HER2 FISH pharmDx Kit, THXID BRAF Kit, Vysis ALK Break Apart FISH Probe Kit, cobas 4800 BRAF V600 Mutation Test, VENTANA PD-L1 (SP142) Assay, *therascreen* FGFR RGQ RT-PCR Kit, and *therascreen* PIK3CA RGQ PCR Kit.

It is contemplated herein that after treatment with a negatively charged particle described herein, optionally in combination with a cancer therapeutic, the level of one or more of biomarkers increases by an amount in the range of from about 1.1 fold to about 10 fold, e.g., about 1.1, about 1.5, about 2, about 2.5, about 3, about 3.5, about 4, about 4.5, about 5, about 5.5, about 6, about 6.5, about 7, about 7.5, about 8, about 8.5, about 9, about 9.5, or about 10 fold. After treatment with a surface functionalized particle described herein, the level of one or more of the biomarkers decrease by an amount in the range of from about 1.1 fold to about 10 fold, e.g., about 1.1, about 1.5, about 2, about 2.5, about 3, about 3.5, about 4, about 4.5, about 5, about 5.5, about 6, about 6.5, about 7, about 7.5, about 8, about 8.5, about 9, about 9.5, or about 10 fold.

Exemplary diseases, conditions or disorders that can be treated using the methods herein include cancers, such as esophageal cancer, pancreatic cancer, metastatic pancreatic cancer, metastatic adenocarcinoma of the pancreas, bladder cancer, stomach cancer, fibrotic cancer, glioma, malignant glioma, diffuse intrinsic pontine glioma, recurrent childhood brain neoplasm renal cell carcinoma, clear-cell metastatic renal cell carcinoma, kidney cancer, prostate cancer, metastatic castration resistant prostate cancer, stage IV prostate cancer, metastatic melanoma, melanoma, malignant melanoma, recurrent melanoma of the skin, melanoma brain metastases, stage IIIA skin melanoma; stage IIIB skin melanoma, stage IllC skin melanoma; stage IV skin melanoma, malignant melanoma of head and neck, lung cancer, non small cell lung cancer (NSCLC), squamous cell non-small cell lung cancer, breast cancer, recurrent metastatic breast cancer, hepatocellular carcinoma, hodgkin's lymphoma, follicular lymphoma, non-hodgkin's lymphoma, advanced B-cell NHL, HL including diffuse large B-cell lymphoma (DLBCL), multiple myeloma, chronic myeloid leukemia, adult acute myeloid leukemia in remission; adult acute myeloid leukemia with Inv(16)(p13.1q22); CBFB-MYH11; adult acute myeloid leukemia with t(16;16)(p13.1;q22); CBFB-MYH11; adult acute myeloid leukemia with t(8;21)(q22;q22); RUNX1-RUNX1T1; adult acute myeloid leukemia with t(9;11)(p22;q23); MLLT3-MLL; adult acute promyelocytic leukemia with t(15;17)(q22;q12); PML-RARA; alkylating agent-related acute myeloid leukemia, chronic lymphocytic leukemia, richter's syndrome; waldenstrom macroglobulinemia, adult glioblastoma; adult gliosarcoma, recurrent glioblastoma, recurrent childhood rhabdomyosarcoma, recurrent Ewing sarcoma/ peripheral primitive neuroectodermal tumor, recurrent neuroblastoma; recurrent osteosarcoma, colorectal cancer, MSI positive colorectal cancer; MSI negative colorectal cancer, nasopharyngeal nonkeratinizing carcinoma; recurrent nasopharyngeal undifferentiated carcinoma, cervical adenocarcinoma; cervical adenosquamous carcinoma; cervical squamous cell carcinoma; recurrent cervical carcinoma; stage IVA cervical cancer; stage IVB cervical cancer, anal canal squamous cell carcinoma; metastatic anal canal carcinoma; recurrent anal canal carcinoma, recurrent head and neck cancer; carcinoma, squamous cell of head and neck, head and neck squamous cell carcinoma (HNSCC), ovarian carcinoma, colon cancer, colorectal cancer, rectal cancer, gastric cancer, advanced GI cancer, gastric adenocarcinoma; gastroesophageal junction adenocarcinoma, bone neoplasms, soft tissue sarcoma; bone sarcoma, thymic carcinoma, urothelial carcinoma, recurrent merkel cell carcinoma; stage III merkel cell carcinoma; stage IV merkel cell carcinoma, myelodysplastic syndrome and recurrent mycosis fungoides and Sezary syndrome. In various embodiments, the cancers are selected from brain cancer, skin cancer, eye cancer, breast cancer, prostate cancer, lung cancer, esophageal cancer, head and neck cancer, cervical cancer, liver cancer, bone cancer, uterine cancer, ovarian cancer, bladder cancer, stomach cancer, oral cancer, thyroid cancer, kidney cancer, testicular cancer, leukemia, lymphoma and mesothelioma.

It is contemplated that the methods herein reduce tumor size or tumor burden in the subject, and/or reduce metastasis in the subject. In various embodiments, the methods reduce the tumor size by 10%, 20%, 30% or more. In various embodiments, the methods reduce tumor size by 10%, 15%, 20%, 25%, 30%, 35%, 40%, 45%, 50%, 55%, 60%, 65%, 70%, 75%, 80%, 85%, 90%, 95% or 100%, including all values and ranges that lie in between these values.

In various embodiments, the disclosure provides a method of reducing the number of immunosuppressive cells from the tumor site by sequestering them in the spleen and/or liver, and inducing apoptosis in a subject comprising administering to the subject negatively charged particles in combination with a cancer therapeutic.

In various embodiments, the administration of the particles in a subject prevents the accumulation of pathology causing monocytes, macrophages, granulocytes and/or neutrophils at the tumor site or tumor microenvironment.

In various embodiments, the disclosure provides a method of reducing the number of monocytes, macrophages, granulocytes and/or neutrophils at the tumor. In various embodiments, the number of monocytes, macrophages, granulocytes and/or neutrophils at the tumor is reduced by about 2-, 3-, 4-, 5-, 6-, 7-, 8-, 9-, or 10-fold or more.

In various embodiments, the disclosure provides a method of altering the anti-tumor immune response in a subject, comprising administering to a subject negatively charged particles in combination with a cancer therapeutic, as defined in the claims. In various embodiments, the altering increases levels or activity of anti-tumor APCs, macrophages, dendritic cells, T-cells, B-cells, NK T-cells, and/or NK cells.

In various embodiments, the administration of the particles in a subject alters the activity and/or function of immune cells at the tumor site or tumor microenvironment. In various embodiments, the administration of the particles increases the number of inflammatory immune cells at the tumor site or tumor microenvironment. In various embodiments, the administration of the particles in the subject increases the anti-tumor inflammatory function or activity of immune cells at the tumor site or tumor microenvironment.

In various embodiments, the disclosure provides a method of altering the tumor-associated stroma, comprising administering to a subject negatively charged particles in combination with a cancer therapeutic.

In various embodiments, the administration of the particles in a subject alters fibroblasts, cancer-associated fibroblasts, adipocytes, endothelial cells, pericytes, mesenchymal stromal cells, and/or ECM at the tumor site or tumor-associated stroma.

In various embodiments, the disclosure provides a method of reducing tumor size and/or tumor growth in a subject, comprising administering to a subject negatively charged particles in combination with a cancer therapeutic.

In various embodiments, the disclosure provides a method of altering cancer stem cells and/or mesenchymal stem cells, comprising administering to a subject negatively charged particles in combination with a cancer therapeutic.

In various embodiments, the administration of the particles in a subject induces tumor-cell death, tumor-cell apoptosis, and/or tumor cell-necrosis via direct particle uptake.

### Administration and Dosing

Contemplated herein are methods comprising administering a negatively charged particle as described herein in combination with a cancer therapeutic, as defined in the claims, to treat a subject suffering from cancer.

Methods of the disclosure are performed using any medically-accepted means for introducing a therapeutic directly or indirectly into a mammalian subject, including but not limited to injections, oral ingestion, intranasal, topical, transdermal, parenteral, inhalation spray, vaginal, or rectal administration. The term parenteral as used herein includes subcutaneous, intravenous, intramuscular, intraarticular, intraperitoneal, intrathecal and intracisternal injections, as well as catheter or infusion techniques. In various embodiments, the particle is administered intravenously, but may be administered through other routes of administration such as, but not limited to: intradermal, subcutaneous, epictuaneous, oral, intra-articular, and intrathecal. In various embodiments, the compositions are administered at the site of the tumor.

In various embodiments, the particle is administered at a dose from about 0.1 to about 10 mg/kg. In various embodiments, the particle is administered at a dose of about 0.1 mg/kg, 0.5 mg/kg, 1.0 mg/kg, 2.0 mg/kg, 4.0 mg/kg, 8.0 mg/kg or 10 mg/kg. In various embodiments, the particle is administered at a dose of about 8.0 mg, 80 mg, 320 mg, 640 mg or 800 mg. Also contemplated are values within and between the recited dose endpoints. These concentrations may be administered as a single dosage form or as multiple doses.

It is contemplated that the cancer therapeutic, if a known cancer therapeutic, is administered as directed by the manufacturer and the treating physician. If the particle and cancer therapeutic are to be administered in the same formulation, they can be formulated as described herein.

The amounts of immune modulator or biologic agent cancer therapeutic in a given dosage may vary according to the size of the individual to whom the therapy is being administered as well as the characteristics of the disorder being treated. In exemplary treatments, it may be necessary to administer about 1 mg/day, 5 mg/day, 10 mg/day, 20 mg/day, 50 mg/day, 75 mg/day, 100 mg/day, 150 mg/day, 200 mg/day, 250 mg/day, 500 mg/day or 1000 mg/day. Standard dose-response studies, first in animal models and then in clinical testing, reveals optimal dosages for particular disease states and patient populations.

In various embodiments, a a PD-1 antibody, is administered in a dose range from 0.1 to 15 mg/kg. These concentrations may be administered as a single dosage form or as multiple doses.

The conditions treatable by methods of the present disclosure preferably occur in mammals. Mammals include, for example, humans and other primates, as well as pet or companion animals such as dogs and cats, laboratory animals such as rats, mice and rabbits, and farm animals such as horses, pigs, sheep, and cattle. In various embodiments, the subject is human.

In various embodiments, the particle is administered twice weekly, once weekly, once every two weeks, once every three weeks, once every 4 weeks, once every two months, once every three months, once every 6 months or once per year.

The disclosure further contemplates a sterile pharmaceutical composition comprising a particle as described herein, a cancer therapeutic and a pharmaceutically acceptable carrier.

The disclosure further contemplates a sterile pharmaceutical composition comprising a particle as described herein and a pharmaceutically acceptable carrier.

The disclosure further contemplates a sterile pharmaceutical composition comprising a cancer therapeutic and a pharmaceutically acceptable carrier.

Syringes, e.g., single use or pre-filled syringes, sterile sealed containers, e.g. vials, bottle, vessel, and/or kits or packages comprising any of the foregoing antibodies or compositions, optionally with suitable instructions for use, are also contemplated.

### Combination Therapy

It is contemplated that the particle described herein is administered in combination with an anti-PD-1 antibody to treat cancer.

Antibodies to PD-1 include Pembrolizumab (KEYTRUDA^{®}, Merck Sharp & Dohme Corp) and nivolumab (OPDIVO^{®}, Bristol-Myers Squibb).

It is contemplated that the particle and the cancer therapeutic can be given concurrently, simultaneously, or sequentially. Concurrent administration of two therapeutic agents does not require that the agents be administered at the same time or by the same route, as long as there is an overlap in the time period during which the agents are exerting their therapeutic effect. Simultaneous or sequential administration is contemplated, as is administration on different days or weeks.

It is contemplated that the particle and the cancer therapeutic may be given simultaneously, in the same formulation. It is further contemplated that the agents are administered in a separate formulation and administered concurrently, with concurrently referring to agents given within 30 minutes of each other.

In another aspect, the cancer therapeutic is administered prior to administration of the particle composition. Prior administration refers to administration of the cancer therapeutic within the range of one week prior to treatment with the particle, up to 30 minutes before administration of the particle. It is further contemplated that the cancer therapeutic is administered subsequent to administration of the particle composition. Subsequent administration is meant to describe administration from 30 minutes after particle treatment up to one week after administration.

### Kits

As an additional aspect, the disclosure includes kits which comprise one or more compounds or compositions packaged in a manner which facilitates their use to practice methods of the disclosure. In one embodiment, such a kit includes a compound or composition described herein (e.g., a composition comprising a particle alone or in combination with a cancer therapeutic), packaged in a container such as a sealed bottle or vessel, with a label affixed to the container or included in the package that describes use of the compound or composition in practicing the method. Preferably, the compound or composition is packaged in a unit dosage form. The kit may further include a device suitable for administering the particle, cancer therapeutic, or composition according to a specific route of administration or for practicing a screening assay. Preferably, the kit contains a label that describes use of the inhibitor compositions.

Additional aspects and details of the disclosure will be apparent from the following examples, which are intended to be illustrative rather than limiting.

### EXAMPLES

### Example 1

### Therapeutic targeting of tumors using IMP therapy in combination with the immune checkpoint inhibitor anti-PD-1 monoclonal antibody

In order to determine the anti-tumor efficacy of IMP therapy in combination with the PD-1 monoclonal antibody (mAb), orthotopic or syngeneic tumor models will be established using 6-8-week old C57BL/6 mice as described by Kumar et al. (Cancer Cell 32, 654-668, (2017])). For Example, mice will be implanted with LLC (Lewis Lung Carcinoma), MC38 or EL4 (9,10-dimethyl-1,2-benzanthracene-induced murine thymoma) cells by s.c injection and divided randomly into 4 treatment groups each, as described below:
Group 1: Control treatment (n=7)
Group 2: IMPs only (n=10)
Group 3: Anti-PD1 mAb only (n=10)
Group 4: IMPs + Anti-PD1 mAb (n=7)

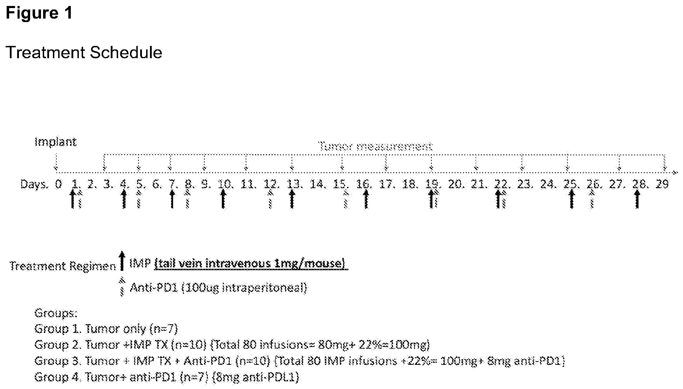

24 hours after s.c. injection with tumor cells, mice are treated with IMPs (1 mg i.v.) and/or anti-PD1 mAb (100 µg i.p.). The following treatment schedule is followed and is illustrated in Figure 1:
Day 0: s.c. injection with LLC cells or EL4 cells.

| **Group** | **Treatment** | **IMP Treatment Days** | **Anti-PD1 mAb Treatment Days** |
|---|---|---|---|
| **1** | Saline (Control) | - | - |
| **2** | IMPs only | 1, 4, 7, 10, 13, 16, 19, 22, 25, 28 | - |
| **3** | Anti-PD1 mAb | - | 11, 14, 17, 20, 23, 26, 29 |
| **4** | IMPs + anti-PD1 mAb | 1, 4, 7, 10, 13, 16, 19, 22, 25, 28 | 11, 14, 17, 20, 23, 26, 29 |

Mice receiving control treatment are given saline i.v. and/or i.p instead of IMPs and anti-PD1 mAb. Tumor growth in each treatment group is assessed by measuring tumor area using standard calipers at days 0, 14, 19, 22, 25 and 29.

Mice receiving saline treatment develop large tumors ranging from 500-600 mm² in area by the end of the treatment period, such that euthanasia is warranted. Conversely, combination therapy with both IMPs and anti-PD1 mAb is expected to result in reduction of, to complete abrogation of, tumor growth. Tumors in the combination treatment group may grow to only 5-10 mm² in area, out to 40 days.

Mice treated with IMPs or anti-PD1 mAb alone are expected to show an intermediate effect with significant delays in tumor growth compared to saline-treated mice. It is hypothesized that tumors ranging from 100-150 mm² in area are expected to be seen toward the end of treatment in these groups. Tumors in these treatment groups may grow to 200-250 mm² in area but are not expected to reach the size of tumors seen in saline-treated mice

In an initial experiment, mice were inoculated with LLC tumor cells via s.c injection at Day 0. LLC tumor cells (ATCC^{®}) were maintained in a monolayer culture in DMEM (Corning CellGro^{®}, 10-013-CV) supplemented with 10% FBS (Atlanta Biologicals), 5nM glutamine, 25 mM HEPES and 1% antibiotics (Invitrogen). A suspension of 0.5 x 10⁶ LLC cells was prepared and was injected into the right lower flank of 7-10-week-old C57BL/6 mice. At Day 1, mice were randomized into treatment groups and treated according to the following schedule. Tumor growth in each treatment group was assessed by measuring tumor area using standard calipers at days 0, 14, 19, 22, 25 and 29. IMPs were PLGA particles.

| **Group** | **Treatment** | **IMP Treatment Days** | **Anti-PD1 mAb Treatment Days** |
|---|---|---|---|
| **1** | Saline (Control) | - | - |
| **2** | IMPs only | 1, 4, 7, 10, 13, 16, 19, 22, 25, 28 | - |
| **3** | Anti-PD1 mAb | - | 11, 14, 17, 20, 23, 26, 29 |
| **4** | IMPs + anti-PD1 mAb | 1, 4, 7, 10, 13, 16, 19, 22, 25, 28 | 11, 14, 17, 20, 23, 26, 29 |

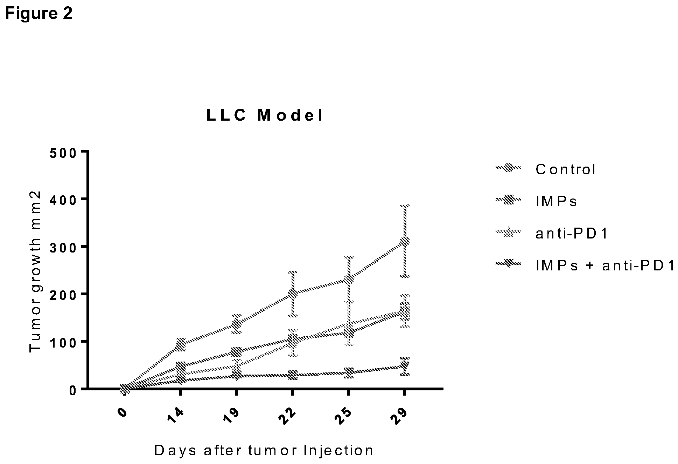

Results are shown in Figure 2. As expected, saline treated (control) mice developed large tumors by day 29. Monotherapy with anti-PD1 or IMPs alone showed comparable anti-tumor effects with a medial inhibition of tumor growth compared to saline treatment. Combination therapy with IMPs and anti-PD1 mAb resulted in a synergistic effect and enhanced inhibition of tumor growth compared to saline treatment or monotherapy with IMPs or anti-PD-1 mAb.

### Example 2

### Therapeutic effect of IMPs administered alone or in combination with anti-PD1

To determine the effects of IMP combination treatment on a syngeneic tumor model, 6-8-week old C57BL/6 mice were implanted with MC38 (colon adenocarcinoma) tumor cells via subcutaneous injection into the flanks. MC38 mouse tumor cells were maintained in a monolayer culture in DMEM supplemented with 10% heat-inactivated fetal bovine serum (FBS) and 2mM L-glutamine at 37°C in a tissue-culture incubator with 5% CO₂. After palpable tumor formation (~100mm³ in size), animals were randomized into one of four treatment groups as follows:
**Group 1:** Control treatment (n=10)
**Group 2:** IMPs (n=10)
**Group 3:** anti-PD1 (n=10)
**Group 4:** IMPs + anti-PD1 (n=10)

A single cell suspension of 1x10⁶ 95% viable cells resuspended in 0.1 mL serum-free DMEM was prepared and injected into the right lower flank of the animals. Treatment was started after tumor sizes reached a size of approximately 100 mm³. IMPs (PLGA particles) (1 mg) were administered via intravenous (i.v) injection and anti-PD-1 (100 µg) was administered via intraperitoneal (i.p) injection according to the following treatment schedule:

| **Group** | **Treatment** | **IMP Treatment Days (After palpable tumor formation)** | **Anti-PD1 mAb Treatment Days (After palpable tumor formation)** |
|---|---|---|---|
| **1** | Control (Saline) | - | - |
| **2** | IMPs | 1, 4, 7, 10, 13, 16, 19, 22, 25 | - |
| **3** | Anti-PD1 mAb | - | 1, 5, 8, 12, 15, 19, 22 |
| **4** | IMPs + anti-PD1 mAb | 1, 4, 7, 10, 13, 16, 19, 22, 25 | 1, 5, 8, 12, 15, 19, 22 |

Tumor growth was evaluated by measuring tumor size in two dimensions using a caliper. Tumor volumes were calculated using the formula V = 0.5 x a x b², where a and b are the long and short diameters of the tumor, respectively. Tumor sizes were expressed in mm³.

As shown in Figure 3A, treatment with IMPs led to a strong inhibition of tumor growth compared to control treatment. IMP efficacy was comparable to anti-PD1 treatment. Treatment with IMPs and anti-PD1 in combination demonstrated synergy and led to increased inhibition of tumor growth compared to respective monotherapies. Reflective of its effect on tumor growth, treatment with IMPs led to prolonged survival of MC38 tumor-bearing mice. IMP survival efficacy was superior to anti-PD1 and combination therapy with IMP and anti-PD1 demonstrated a synergistic effect that resulted in enhanced survival when compared to respective monotherapies (Figure 3B).

## Claims

1. Negatively charged poly (lactic-co-glycolic acid) (PLGA) particles for use in a method of treating cancer in a subject, the method comprising administering the negatively charged particles in combination with a cancer therapeutic, wherein the negatively charged particles are biodegradable particles that are free of associated peptides or antigenic moieties or other bioactive agents, wherein the cancer therapeutic is an anti-PD-1 antibody.

2. A cancer therapeutic for use in a method of treating cancer in a subject, the method comprising administering negatively charged poly (lactic-co-glycolic acid) (PLGA) particles in combination with the cancer therapeutic, wherein the negatively charged particles are biodegradable particles that are free of associated peptides or antigenic moieties or other bioactive agents, wherein the cancer therapeutic is an anti-PD-1 antibody.

3. The negatively charged particles for use according to claim 1, or the cancer therapeutic for use according to claim 2, wherein administering the negatively charged particles and cancer therapeutic alters myeloid-derived suppressor cells (MDSCs), tumor-associated macrophages (TAMs), neutrophils, monocytes, cancer stem cells, mesenchymal stem cells, fibroblasts, adipocytes, endothelial cells, mesenchymal stromal cells, and/or extracellular matrix in the tumor microenvironment.

4. The negatively charged particles or the cancer therapeutic for use according to any one of the preceding claims, wherein the negatively charged particles are carboxylated.

5. The negatively charged particles or the cancer therapeutic for use according to any one of the preceding claims, wherein the PLGA particles comprise about 50:50, about 80:20 to about 100:0 polylactic acid:polyglycolic acid or from about 50:50, about 80:20 to about 100:0 polyglycolic acid:polylactic acid.

6. The negatively charged particles or the cancer therapeutic for use according to any one of the preceding claims, wherein the particle has a zeta potential between -100 mV and -1 mV, and optionally, between -80 mV and -30 mV.

7. The negatively charged particles or the cancer therapeutic for use according to any one of the preceding claims, wherein the diameter of the negatively charged particle is between 0.1 µm to 10 µm, and optionally, between 400 nm to 800 nm.

8. The negatively charged particles or the cancer therapeutic for use according to any one of the preceding claims, wherein the subject has a brain cancer, skin cancer, eye cancer, breast cancer, pancreatic cancer, prostate cancer, lung cancer, esophageal cancer, head and neck cancer, cervical cancer, liver cancer, colon cancer, colorectal cancer, rectal cancer, bone cancer, uterine cancer, ovarian cancer, bladder cancer, stomach cancer, oral cancer, thyroid cancer, kidney cancer, testicular cancer, leukemia, lymphoma or mesothelioma.

9. The negatively charged particles or the cancer therapeutic for use according to any one of the preceding claims, wherein the immune checkpoint modulator is pembrolizumab or nivolumab.

10. The negatively charged particles or the cancer therapeutic for use according to any one of the preceding claims wherein the particle and/or the cancer therapeutic is administered twice weekly, once weekly, once every two weeks, once every three weeks, once every 4 weeks, once every two months, once every three months, once every 6 months or once per year.

11. The negatively charged particles or the cancer therapeutic for use according to any one of the preceding claims, wherein the particles are administered intravenously, orally, nasally, intramuscularly, ocularly, transdermally, or subcutaneously.

12. The negatively charged particles or the cancer therapeutic for use according to any one of the preceding claims, wherein the subject is human.

13. The negatively charged particles or the cancer therapeutic for use according to any one of the preceding claims, wherein the administration improves one or more symptoms of the cancer, wherein the one or more symptoms are selected from the group consisting of tumor size or tumor burden in the subject, tumor metastasis, and levels of inflammatory cells in the tumor.

14. The negatively charged particles or the cancer therapeutic for use according to any one of the preceding claims, wherein the particle and/or cancer therapeutic is formulated in a composition comprising a pharmaceutical acceptable carrier, diluent or excipient.

15. The negatively charged particles or the cancer therapeutic for use according to any one of the preceding claims, wherein the administration regulates an anti-tumor immune response in the subject.

## Patentansprüche

1. Negativ geladene Poly(lactid-co-glycolid)(PLGA)-Partikel zur Verwendung in einem Verfahren zum Behandeln von Krebs bei einem Subjekt, wobei das Verfahren Verabreichen der negativ geladenen Partikel in Kombination mit einem Krebstherapeutikum umfasst, wobei die negativ geladenen Partikel biologisch abbaubare Partikel sind, die frei von assoziierten Peptiden oder antigenen Einheiten oder anderen bioaktiven Mitteln sind, wobei das Krebstherapeutikum ein Anti-PD-1-Antikörper ist.

2. Krebstherapeutikum zur Verwendung in einem Verfahren zum Behandeln von Krebs bei einem Subjekt, wobei das Verfahren Verabreichen von negativ geladenen Poly(lactid-co-glycolid)-Partikeln in Kombination mit dem Krebstherapeutikum umfasst, wobei die negativ geladenen Partikel biologisch abbaubare Partikel sind, die frei von assoziierten Peptiden oder antigenen Einheiten oder anderen bioaktiven Mitteln sind, wobei das Krebstherapeutikum ein Anti-PD-1-Antikörper ist.

3. Negativ geladene Partikel zur Verwendung nach Anspruch 1 oder Krebstherapeutikum zur Verwendung nach Anspruch 2, wobei das Verabreichen der negativ geladenen Partikel und des Krebstherapeutikums myeloide Suppressorzellen (MDSCs), tumorassoziierte Makrophagen (TAMs), Neutrophile, Monozyten, Krebsstammzellen, mesenchymale Stammzellen, Fibroblasten, Adipozyten, Endothelzellen, mesenchymale Stromazellen und/oder extrazelluläre Matrix in der Tumormikroumgebung verändert.

4. Negativ geladene Partikel oder Krebstherapeutikum zur Verwendung nach einem der vorhergehenden Ansprüche, wobei die negativ geladenen Partikel carboxyliert sind.

5. Negativ geladene Partikel oder Krebstherapeutikum zur Verwendung nach einem der vorhergehenden Ansprüche, wobei die PLGA-Partikel etwa 50:50, etwa 80:20 bis etwa 100:0 Polymilchsäure:Polyglycolsäure oder von etwa 50:50, etwa 80:20 bis etwa 100:0 Polyglycolsäure:Polymilchsäure umfassen.

6. Negativ geladene Partikel oder Krebstherapeutikum zur Verwendung nach einem der vorhergehenden Ansprüche, wobei der Partikel ein Zeta-Potential zwischen -100 mV und -1 mV und gegebenenfalls zwischen -80 mV und -30 mV aufweist.

7. Negativ geladene Partikel oder Krebstherapeutikum zur Verwendung nach einem der vorhergehenden Ansprüche, wobei der Durchmesser des negativ geladenen Partikels zwischen 0,1 µm und 10 µm und gegebenenfalls zwischen 400 nm und 800 nm liegt.

8. Negativ geladene Partikel oder Krebstherapeutikum zur Verwendung nach einem der vorhergehenden Ansprüche, wobei das Subjekt Gehirnkrebs, Hautkrebs, Augenkrebs, Brustkrebs, Pankreaskrebs, Prostatakrebs, Lungenkrebs, Speiseröhrenkrebs, Kopf- und Halskrebs, Gebärmutterhalskrebs, Leberkrebs, Darmkrebs, Dickdarmkrebs, Rektalkrebs, Knochenkrebs, Gebärmutterkrebs, Eierstockkrebs, Blasenkrebs, Magenkrebs, Mundhöhlenkrebs, Schilddrüsenkrebs, Nierenkrebs, Hodenkrebs, Leukämie, ein Lymphom oder ein Mesotheliom aufweist.

9. Negativ geladene Partikel oder Krebstherapeutikum zur Verwendung nach einem der vorhergehenden Ansprüche, wobei der Immun-Checkpoint-Modulator Pembrolizumab oder Nivolumab ist.

10. Negativ geladene Partikel oder Krebstherapeutikum zur Verwendung nach einem der vorhergehenden Ansprüche, wobei der Partikel und/oder das Krebstherapeutikum zweimal wöchentlich, einmal wöchentlich, einmal alle zwei Wochen, einmal alle drei Wochen, einmal alle vier Wochen, einmal alle zwei Monate, einmal alle drei Monate, einmal alle sechs Monate oder einmal pro Jahr verabreicht wird.

11. Negativ geladene Partikel oder Krebstherapeutikum zur Verwendung nach einem der vorhergehenden Ansprüche, wobei die Partikel intravenös, oral, nasal, intramuskulär, okular, transdermal oder subkutan verabreicht werden.

12. Negativ geladene Partikel oder Krebstherapeutikum zur Verwendung nach einem der vorhergehenden Ansprüche, wobei das Subjekt menschlich ist.

13. Negativ geladene Partikel oder Krebstherapeutikum zur Verwendung nach einem der vorhergehenden Ansprüche, wobei die Verabreichung ein oder mehrere Symptome der Krebserkrankung verbessert, wobei das eine oder die mehreren Symptome ausgewählt sind aus der Gruppe bestehend aus Tumorgröße oder Tumorlast in dem Subjekt, Tumormetastasen und Spiegel von Entzündungszellen in dem Tumor.

14. Negativ geladene Partikel oder Krebstherapeutikum zur Verwendung nach einem der vorhergehenden Ansprüche, wobei der Partikel und/oder das Krebstherapeutikum in einer Zusammensetzung formuliert ist, die einen pharmazeutisch verträglichen Träger, Verdünnungsstoff oder Hilfsstoff umfasst.

15. Negativ geladene Partikel oder Krebstherapeutikum zur Verwendung nach einem der vorhergehenden Ansprüche, wobei die Verabreichung eine Antitumor-Immunantwort in dem Subjekt reguliert.

## Revendications

1. Particules de poly(acide lactique-co-glycolique) (PLGA) chargées négativement pour une utilisation dans un procédé de traitement du cancer chez un sujet, le procédé comprenant l'administration des particules chargées négativement en combinaison avec un agent thérapeutique contre le cancer, dans lesquelles les particules chargées négativement sont des particules biodégradables qui sont exemptes de peptides associés ou de fractions antigéniques ou d'autres agents bioactifs, dans lesquelles l'agent thérapeutique contre le cancer est un anticorps anti-PD-1.

2. Agent thérapeutique contre le cancer pour une utilisation dans un procédé de traitement du cancer chez un sujet, le procédé comprenant l'administration de particules de poly(acide lactique-co-glycolique) (PLGA) chargées négativement en combinaison avec l'agent thérapeutique contre le cancer, dans lequel les particules chargées négativement sont des particules biodégradables qui sont exemptes de peptides associés ou de fractions antigéniques ou d'autres agents bioactifs, dans lequel l'agent thérapeutique contre le cancer est un anticorps anti-PD-1.

3. Particules chargées négativement pour une utilisation selon la revendication 1, ou agent thérapeutique contre le cancer pour une utilisation selon la revendication 2, dans lesquels l'administration des particules chargées négativement et de l'agent thérapeutique contre le cancer modifie les cellules suppressives d'origine myéloïde (MDSC), les macrophages associés aux tumeurs (TAM), les neutrophiles, les monocytes, les cellules souches cancéreuses, les cellules souches mésenchymateuses, les fibroblastes, les adipocytes, les cellules endothéliales, les cellules stromales mésenchymateuses, et/ou la matrice extracellulaire dans le microenvironnement tumoral.

4. Particules chargées négativement ou agent thérapeutique contre le cancer pour une utilisation selon l'une quelconque des revendications précédentes, dans lesquels les particules chargées négativement sont carboxylées.

5. Particules chargées négativement ou agent thérapeutique contre le cancer pour une utilisation selon l'une quelconque des revendications précédentes, dans lesquels les particules de PLGA comprennent environ 50:50, environ 80:20 à environ 100:0 d'acide polylactique:acide polyglycolique ou provenant d'environ 50:50, environ 80:20 à environ 100:0 d'acide polyglycolique:acide polylactique.

6. Particules chargées négativement ou agent thérapeutique contre le cancer pour une utilisation selon l'une quelconque des revendications précédentes, dans lesquels la particule a un potentiel zêta compris entre -100 mV et -1 mV, et éventuellement, entre -80 mV et -30 mV.

7. Particules chargées négativement ou agent thérapeutique contre le cancer pour une utilisation selon l'une quelconque des revendications précédentes, dans lesquels le diamètre de la particule chargée négativement est compris entre 0,1 µm et 10 µm, et éventuellement, entre 400 nm et 800 nm.

8. Particules chargées négativement ou agent thérapeutique contre le cancer pour une utilisation selon l'une quelconque des revendications précédentes, dans lesquels le sujet présente un cancer du cerveau, un cancer de la peau, un cancer de l'œil, un cancer du sein, un cancer du pancréas, un cancer de la prostate, un cancer du poumon, un cancer de l'œsophage, un cancer de la tête et du cou, un cancer du col de l'utérus, un cancer du foie, un cancer du côlon, un cancer colorectal, un cancer rectal, un cancer des os, un cancer de l'utérus, un cancer de l'ovaire, un cancer de la vessie, un cancer de l'estomac, un cancer buccal, un cancer de la thyroïde, un cancer du rein, un cancer testiculaire, une leucémie, un lymphome ou un mésothéliome.

9. Particules chargées négativement ou agent thérapeutique contre le cancer pour une utilisation selon l'une quelconque des revendications précédentes, dans lesquels le modulateur de point de contrôle immunitaire est le pembrolizumab ou le nivolumab.

10. Particules chargées négativement ou agent thérapeutique contre le cancer pour une utilisation selon l'une quelconque des revendications précédentes, dans lesquels la particule et/ou l'agent thérapeutique contre le cancer est administré deux fois par semaine, une fois par semaine, une fois toutes les deux semaines, une fois toutes les trois semaines, une fois toutes les 4 semaines, une fois tous les deux mois, une fois tous les trois mois, une fois tous les 6 mois ou une fois par an.

11. Particules chargées négativement ou agent thérapeutique contre le cancer pour une utilisation selon l'une quelconque des revendications précédentes, dans lesquels les particules sont administrées par voie intraveineuse, orale, nasale, intramusculaire, oculaire, transdermique ou sous-cutanée.

12. Particules chargées négativement ou agent thérapeutique contre le cancer pour une utilisation selon l'une quelconque des revendications précédentes, dans lesquels le sujet est humain.

13. Particules chargées négativement ou agent thérapeutique contre le cancer pour une utilisation selon l'une quelconque des revendications précédentes, dans lesquels l'administration améliore un ou plusieurs symptômes du cancer, dans lesquels les un ou plusieurs symptômes sont choisis dans le groupe constitué par la taille de la tumeur ou la charge tumorale chez le sujet, les métastases tumorales, et les taux de cellules inflammatoires dans la tumeur.

14. Particules chargées négativement ou agent thérapeutique contre le cancer pour une utilisation selon l'une quelconque des revendications précédentes, dans lesquels la particule et/ou l'agent thérapeutique contre le cancer est formulé dans une composition comprenant un véhicule, un diluant ou un excipient pharmaceutiquement acceptable.

15. Particules chargées négativement ou agent thérapeutique contre le cancer pour une utilisation selon l'une quelconque des revendications précédentes, dans lesquels l'administration régule une réponse immunitaire antitumorale chez le sujet.
